# EUROPEAN PATENT SPECIFICATION

(11) **EP 3 930 839 B1**
(45) Date of publication and mention of the grant of the patent: **28.01.2026**
(21) Application number: 20762779.5
(22) Date of filing: 25.02.2020
(51) Int. Cl.: A61K 31/351, A61K 31/4985, A61P 13/08, A61P 13/02, C07H 15/224, C07H 15/236, A61K 31/519, A61K 45/06, A61K 31/18, A61K 31/4045, A61K 31/517

(54) **ONVANSERTIB FOR INHIBITING NON-ADRENERGIC CONTRACTION OF SMOOTH MUSCLE AND PROSTATE CELL PROLIFERATION**
ONVANSERTIB ZUR HEMMUNG DER NICHT-ADRENERGEN KONTRAKTION VON GLATTEN MUSKELN UND PROSTATAZELLPROLIFERATION
ONVANSERTIB POUR INHIBER LA CONTRACTION NON-ADRÉNERGIQUE D'UN MUSCLE LISSE ET LA PROLIFÉRATION DES CELLULES DE LA PROSTATE

(30) Priority: 25.02.2019 US 201962810171 P
(43) Date of publication of application: 05.01.2022
(73) Proprietor: Cardiff Oncology, Inc., San Diego CA 92121 (US)
(72) Inventor: ERLANDER, Mark, San Diego, CA 92121 (US); RIDINGER, Maya, San Diego, CA 92121 (US); ADAMS, Thomas H., San Diego, California 92121 (US)
(74) Representative: J A Kemp LLP
(86) International application number: PCT/US2020/019647
(87) International publication number: WO 2020/176470

(56) References cited:
- WO-A1-2008/074788
- US-A1- 2004 138 241
- US-A1- 2012 114 641
- US-B2- 9 566 280
- HENNENBERG MARTIN, KUPPERMANN PAUL, YU QINGFENG, HERLEMANN ANNIKA, TAMALUNAS ALEXANDER, WANG YIMING, RUTZ BEATA, CIOTKOWSKA ANNA, : "Inhibition of Prostate Smooth Muscle Contraction by Inhibitors of Polo-Like Kinases", FRONTIERS IN PHYSIOLOGY, vol. 9, 15 June 2018 (2018-06-15), pages 1 - 15, XP055734336
- WANG ET AL.: "Onvansertib, a polo-like kinase 1 inhibitor, inhibits prostate stromal cell growth and . prostate smooth muscle contraction, which is additive to inhibition by alpha1-blockers, European", JOUMAL OF PHARMACOLOGY, vol. 873, 1 February 2020 (2020-02-01), pages 1 - 12, XP086071762, DOI: 10.1016/j.ejphar.2020.172985

## Description

### CROSS-REFERENCE TO RELATED APPLICATIONS

This application claims the benefit of U.S. Provisional Application No. 62/810171, filed February 25, 2019.

### BACKGROUND OF THE INVENTION

### (1) Field of the Invention

The present application generally relates to treatments for benign prostatic hyperplasia (BPH). More specifically, treatment of BPH with highly selective polo-like kinase 1 (PLK1) inhibitors are provided.

### (2) Description of the related art

Benign prostatic hyperplasia (BPH) is commonly associated with lower urinary tract symptoms (LUTS), due to urethral compression resulting from prostate enlargement and increased prostate smooth muscle tone in BPH (Hennenberg et al., 2014). This condition of symptomatic BPH, termed as bladder outlet obstruction (BOO), often requires medical treatment (Oelke et al., 2013). However, this has limited effectiveness even in mild to moderate LUTS, and is not available for severe LUTS (Oelke et al., 2013). The restriction of improvements is contrasted by high case numbers, ranging around 600 million patients with obstructive symptoms worldwide in 2018 (Irwin et al., 2011). Due to the age-dependency of prevalence together with the demographic transition, the importance of LUTS suggestive of BPH will further increase, raising a high demand of new medical therapies with higher effectiveness.

According to the contributions of prostate smooth muscle tone and prostate growth to LUTS suggestive of BPH, medical treatment in BPH aims inhibition 1) of prostate smooth muscle contraction for rapid improvement of symptoms, and 2) of prostate growth to prevent BPH progression and complications (Oelke et al., 2013). Prostate smooth muscle contraction is induced partly by activation of α₁-adrenoceptors by noradrenaline, which is released during adrenergic neurotransmission (Hennenberg et al., 2014). Consequently, α₁-adrenoceptor antagonists ("α₁-blockers") are the first line option for treatment of LUTS suggestive of BPH, as they may reduce BOO by relaxation of prostate smooth muscle (Caine et al., 1975, 1976; Oelke et al., 2013). However, they reduce symptoms (international prostate symptom scores, IPSS) and improve urinary flow (Qₘₐₓ) by not more than 50%, while even placebos may cause improvements up to 30% (Hennenberg et al., 2014, 2017; Oelke et al., 2013; Strand et al., 2017). The phosphodiesterase-5 inhibitor tadalafil has been recently approved for treatment of obstructive LUTS, but its effectiveness is not higher than that of α₁-blockers (Dahm et al., 2017). 5α-reductase inhibitors (5-ARIs) are applied to stop prostate growth, with the aim to reduce prostate size and to prevent BPH progression (Oelke et al., 2013). However, the risk of symptomatic progression can be reduced not more than 35-40% by monotherapy, and 66% by combination therapies (Strand et al., 2017). Disappointing results of medical LUTS therapy contribute to high discontinuation rates, amounting up to 70% of patients who discontinue their medication within 12 month after first prescription (Cindolo et al., 2015). Consequences are disease progression, hospitalization, and surgery for BPH (Cindolo et al., 2015). The limitations of α₁-blockers may result from contributions of nonadrenergic mediators to prostate smooth muscle tone, including endothelin-1, which induce prostate smooth muscle contraction in parallel to α₁-adrenoceptors (Hennenberg et al., 2014, 2017).

Polo-like kinases (PLKs) are serine-threonine kinases, and have been primarily associated with promotion of proliferation in different cell types, including airway smooth muscle cells, prostate cancer cells, and others (Jiang and Tang, 2015; Lin et al., 2019; Shao et al., 2015). In addition, recent findings suggested a function of PLK1 for promotion of airway, vascular, and human prostate smooth muscle contraction (de Carcer et al., 2017; Hennenberg et al., 2018; Li et al., 2016). US 2012/114641 A1 discloses onvaneretib as a PLK1 inhibitor for use in combination with one or more antineoplastic agents in the treatment of a proliferative disorder. However, additive effects of PLK inhibitors to α₁-blockers in prostate smooth muscle contraction, and their effects on growth of prostate stromal cells are unknown.

### Onvansertib

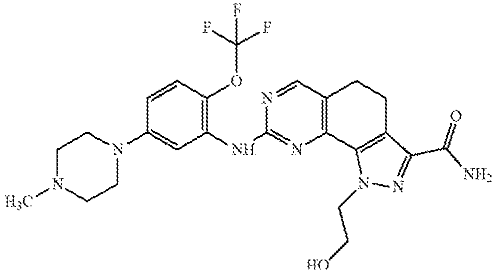

### ONVANSERTIB

Onvansertib (also known as PCM-075, NMS-1286937, NMS-937, "compound of formula (I)" in U.S. Patent 8,927,530; IUPAC name 1-(2-hydroxyethyl)-8-{[5-(4-methylpiperazin-1-yl)-2-(trifluoromethoxy) phenyl] amino}- 4,5-dihydro-1H-pyrazolo[4,3-h] quinazoline-3-carboxamide) is the first PLK1 specific ATP competitive inhibitor administered by oral route to enter clinical trials with proven antitumor activity in different preclinical models. Onvansertib has favorable pharmacologic parameters and good oral bioavailability. Onvansertib has several advantages over other PLK1 inhibitors, including a very high selectivity for the PLK1 isozyme, and oral bioavailability. Onvansertib has an IC₅₀ of 2 nM for PLK1 and >10,000 for PLK2 and PLK3.

### BRIEF SUMMARY OF THE INVENTION

Any references in the description to methods of treatment refer to the compounds, pharmaceutical compositions and medicaments of the invention for use in a method of treatment of the human body by therapy.

Provided is a method of treating benign prostatic hyperplasia (BPH) in a patient. The method comprises treating the patient with (a) onvansertib and (b) an α1-blocker.

### BRIEF DESCRIPTION OF THE SEVERAL VIEWS OF THE DRAWINGS

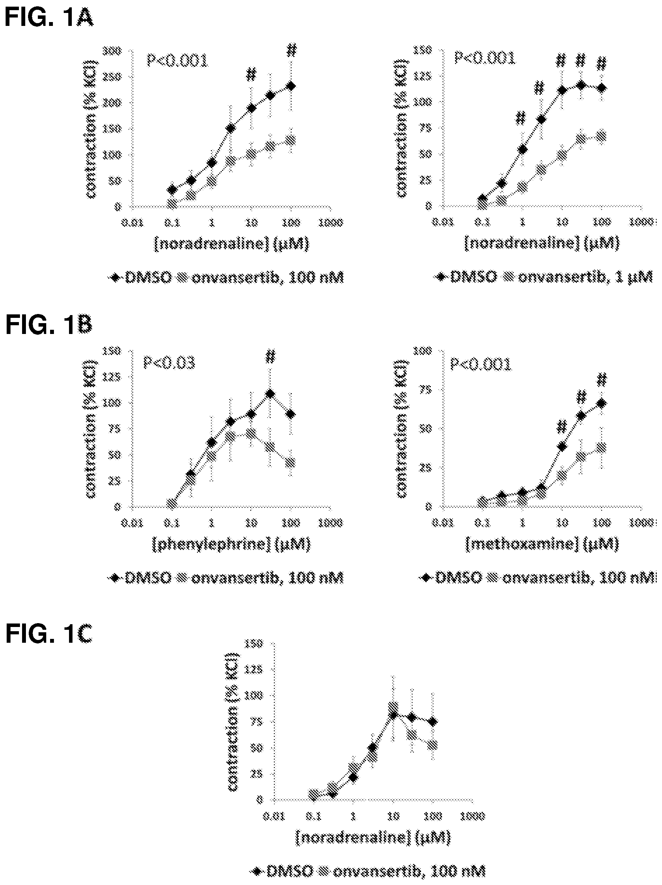

FIGS. 1A, 1B and 1C are graphs showing the effects of onvansertib on adrenergic contractions of human prostate strips. Contractions of human prostate strips were induced by noradrenaline (FIG. 1A), or by the α₁-adrenoceptor agonists phenylephrine and methoxamine (FIG. 1B) in an organ bath, after addition of onvansertib in a concentration of 100 nM or 1 µM, or of DMSO (controls), or induced by noradrenaline (FIG. 1C) following washout of DMSO and onvansertib (100 nM) for 30 min. In each experiment, DMSO and onvansertib were applied to separate samples, which were obtained from the same prostates. To eliminate heterogeneities due to individual variations, different degree of BPH or other varying smooth muscle content, tensions have been expressed as % of contraction by high molar KCl, being assessed before application of onvansertib or DMSO. Data are means ± S.E.M. from series with tissues from n = 5 (noradrenaline with 100 nM onvansertib without washout), n = 6 (noradrenaline with 1 µM onvansertib), n = 5 (phenylephrine), n = 5 (methoxamine), and n = 5 (noradrenaline after washout) patients, in which samples from each patient were allocated to both the control and inhibitor groups (#P < 0.05 after multivariate analysis at indicated concentration; P value for whole groups after two-way ANOVA as indicated in inserts).

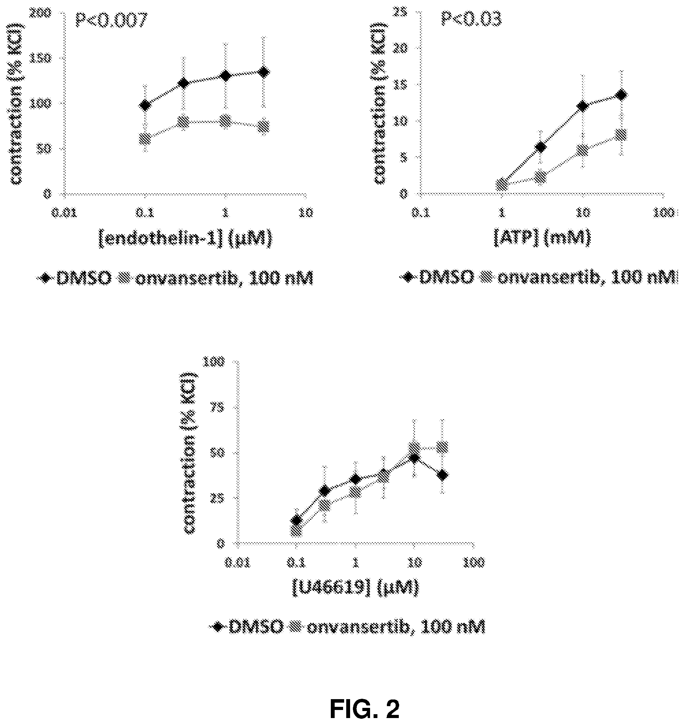

FIG. 2 is graphs showing the effects of onvansertib on nonadrenergic contractions of human prostate strips. Contractions of human prostate strips were induced by endothelin-1, ATP, or the thromboxane A2 analog U46619 in an organ bath, after addition of onvansertib in a concentration of 100 nM, or of DMSO (controls). In each experiment, DMSO and onvansertib were applied to separate samples, which were obtained from the same prostates. To eliminate heterogeneities due to individual variations, different degree of BPH or other varying smooth muscle content, tensions have been expressed as % of contraction by highmolar KCl, being assessed before application of onvansertib or DMSO. Data are means ± S.E.M. from series with tissues from n = 6 (endothelin-1), n = 5 (ATP), and n = 5 (U46619), and patients, in which samples from each patient were allocated to both the control and inhibitor groups (P value for whole groups after two-way ANOVA as indicated in inserts).

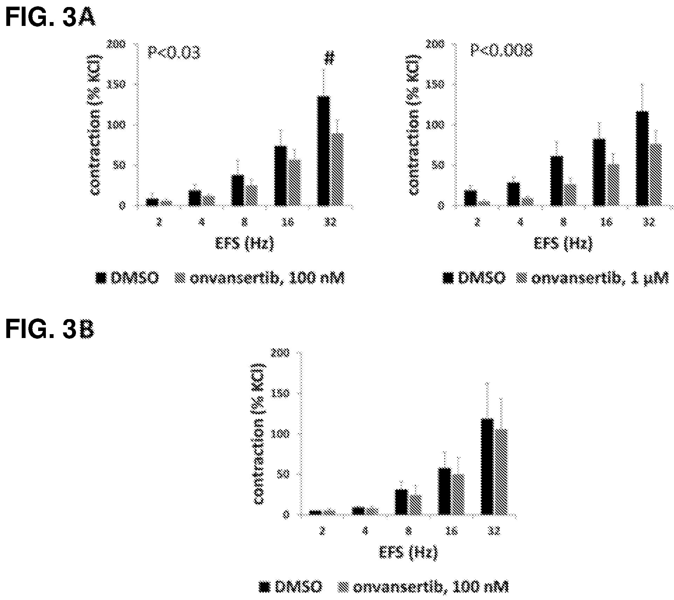

FIGS. 3A and 3B are graphs showing the effects of onvansertib on EFS-induced contractions of human prostate strips. Contractions of human prostate strips were induced by EFS in an organ bath, after addition of onvansertib in a concentration of 100 nM or 1 µM, or of DMSO (controls) (FIG. 3A), and following washout of DMSO and onvansertib (100 nM) for 30 min (FIG. 3B). In each experiment, DMSO and onvansertib were applied to separate samples, which were obtained from the same prostates. To eliminate heterogeneities due to individual variations, different degree of BPH or other varying smooth muscle content, tensions have been expressed as % of contraction by high molar KCl, being assessed before application of onvansertib or DMSO. Data are means ± S.E.M. from series with tissues from n = 6 (DMSO vs. 100 nM onvansertib without washout), n = 7 (DMSO vs. 1 µM onvansertib), and n = 5 (DMSO vs. 100 nM onvansertib after washout) patients, in which samples from each patient were allocated to both the control and inhibitor groups (P value for whole groups after two-way ANOVA as indicated in inserts).

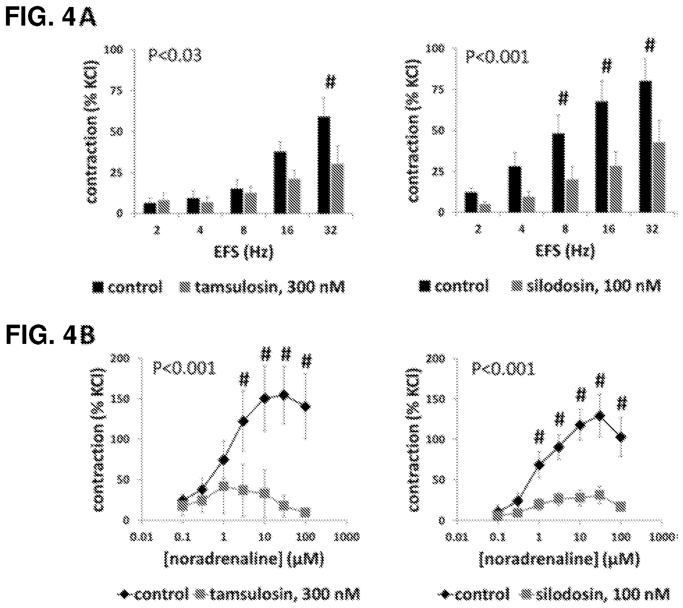

FIGS. 4A and 4B are graphs showing the effects of α₁-blockers on contractions of human prostate strips. Contractions of human prostate strips were induced by EFS (FIG. 3A) or noradrenaline (FIG. 4B) in an organ bath, after addition of tamsulosin (300 nM), silodosin (100 nM), or an equivalent volume of distilled water containing 1% DMSO (controls). In each experiment, solvent or α₁-blockers were applied to separate samples, which were obtained from the same prostates. To eliminate heterogeneities due to individual variations, different degree of BPH or other varying smooth muscle content, tensions have been expressed as % of contraction by high molar KCl, being assessed before application of α₁-blockers or solvent. Data are means ± S.E.M. from series with tissues from n = 5 (EFS/tamsulosin), n = 5 (EFS/silodosin), n = 5 (noradrenaline/tamsulosin), and n = 6 (noradrenaline/silodosin) patients, in which samples from each patient were allocated to both the control and inhibitor groups (#P < 0.05 after multivariate analysis at indicated concentration; P value for whole groups after two-way ANOVA as indicated in inserts).

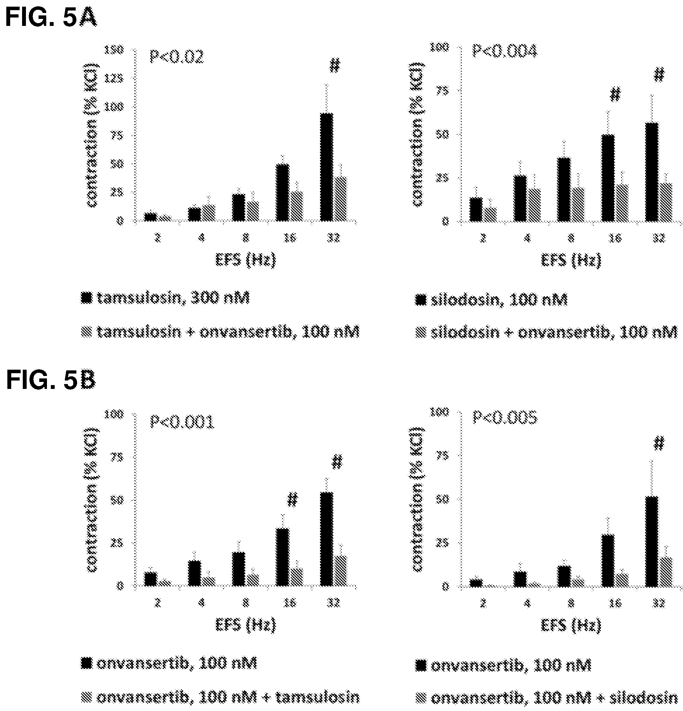

FIGS. 5A and 5B are graphs showing the effects of combinations of onvansertib with α₁-blockers on EFS-induced contractions of human prostate strips, compared to α₁-blockers alone. Contractions of human prostate strips were induced by EFS in an organ bath, after addition of tamsulosin (300 nM), silodosin (100 nM), onvansertib (100 nM), or combinations of onvansertib (100 nM) with tamsulosin or silodosin, and equivalent amounts of solvent (DMSO as control for lacking onvansertib to α₁-blockers alone, or water/DMSO as control for lacking α1-blocker to onvansertib alone). In each experiment, α₁-blockers or onvansertib and combinations were applied to separate samples, which were obtained from the same prostates. Each diagram represents independent series of experiments using different prostate tissues (but with allocation of samples from the same prostates to both groups in each diagram). To eliminate heterogeneities due to individual variations, different degree of BPH or other varying smooth muscle content, tensions have been expressed as % of contraction by high molar KCl, being assessed before application of onvansertib, α₁-blockers, or DMSO. Data are means ± S.E.M. from series with tissues from n = 7 (tamsulosin vs. tamsulosin with onvansertib), n = 5 (silodosin vs. silodosin with onvansertib), n = 5 (onvansertib vs. tamsulosin with onvansertib), and n = 5 (onvansertib vs. silodosin with onvansertib) patients, in which samples from each patient were allocated to both the control and inhibitor groups (#P < 0.05 after multivariate analysis at indicated concentration; P value for whole groups after two-way ANOVA as indicated in inserts).

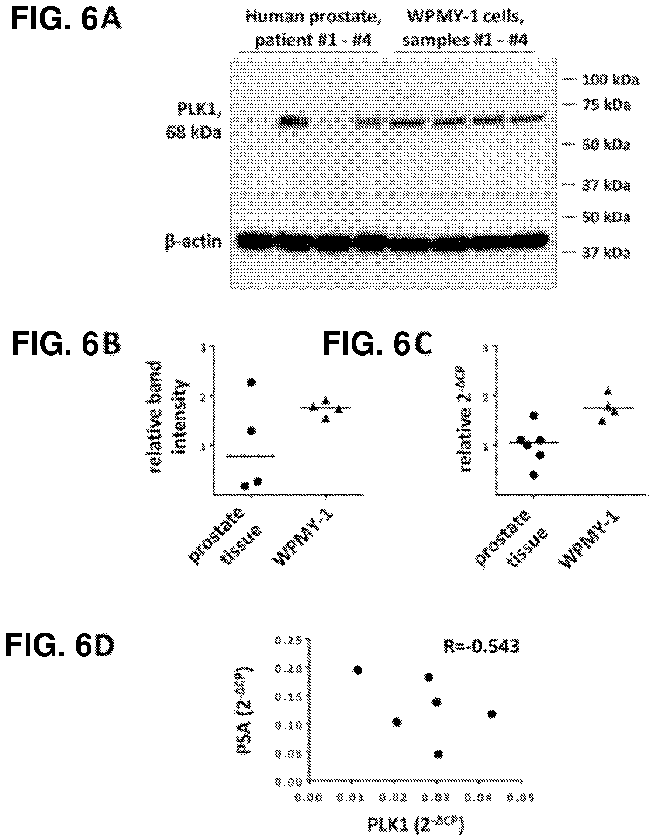

FIGS. 6A, 6B, 6C and 6D are a photograph (FIG. 6A) and graphs showing PLK1 detection in human prostate tissues and WPMY-1 cells. FIG. 6A shows the result of a Western blot analyses performed with human prostate tissues (n = 4 patients) and WPMY-1 cells (samples from n = 4 independent experiments), using an antibody raised against PLK1. Shown are bands with the size of the expected molecular weight of PLK1 (68 kDa). Positions and sizes of marker bands are indicated at the right side of each blot (sizes in kDa). FIG. 6B shows relative intensities of presumed PLK1 bands. Following densitometric quantification of 68 kDa bands in (FIG. 6A), arbitrary units of all samples were normalized to the mean of values obtained for prostate tissues. Shown are values for all samples and the median for both groups. FIG. 6C shows relative mRNA expression of PLK1 in human prostate tissues (n = 6 patients) and WPMY-1 cells (samples from n = 4 independent experiments). Ct values from RT-PCR were expressed as 2^{-ΔCP}, and finally normalized to the mean value obtained for prostate tissues. Shown are values for all samples and the median for both groups. FIG. 6D shows 2^{-ΔCP} values from detection of PLK1 and PSA by RT-PCR in human prostate tissues were plotted against each other, and subjected to Spearman's correlation analysis.

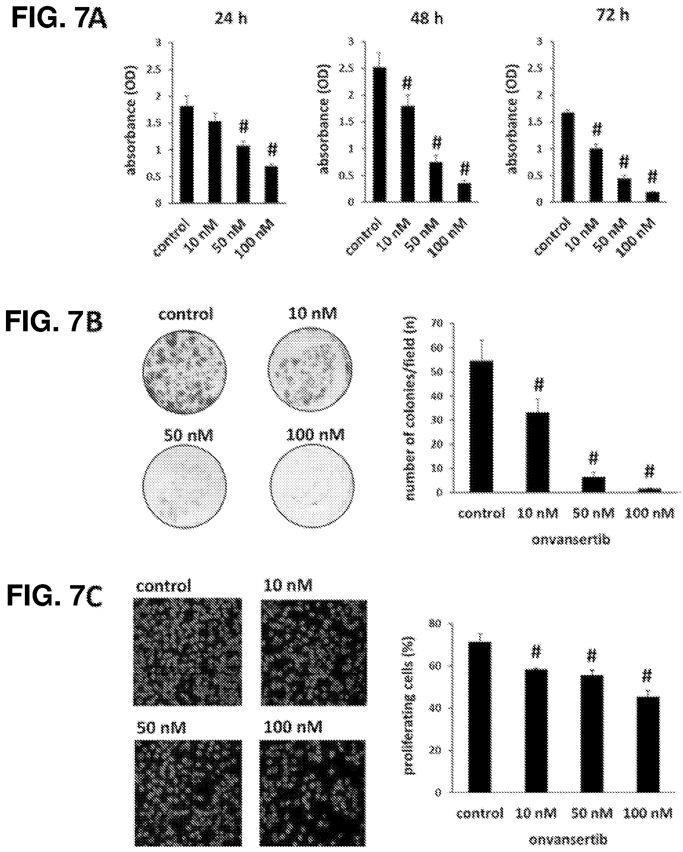

FIGS. 7A, 7B and 7C are graphs and micrographs showing the effects of onvansertib on viability and proliferation of WPMY-1 cells. FIG. 7A is graphs showing viability of WPMY-1 cells was assessed by CCK-8 assay, following exposure to onvansertib or solvent in concentrations and for periods as indicated. Data are means ± S.E.M. from n = 5 independent experiments for each setting (#P < 0.05 vs. corresponding control). FIG. 7B is a graph and micrographs showing proliferation assessed by a colony formation assay. WPMY-1 cells were exposed to onvansertib in indicated concentrations. Shown are representative micrographs, and means ± S.E.M. from n = 5 independent experiments (#P < 0.05 vs. control). FIG. 7C is a graph and micrographs showing proliferation assessed by an EdU assay. WPMY-1 cells were exposed to onvansertib in indicated concentrations for 24 h. Nuclei of proliferating cells are shown in pink, while nuclei of non-proliferating cells are shown in blue. Shown are representative pictures, and means ± S.E.M. from n = 5 independent experiments (#P < 0.05 vs. control).

### DETAILED DESCRIPTION OF THE INVENTION

As used herein, the singular forms "a", "an" and "the" are intended to include the plural forms as well, unless the context clearly indicates otherwise. Additionally, the use of "or" is intended to include "and/or", unless the context clearly indicates otherwise.

The present invention is based in part on the discovery that the highly selective polo-like kinase 1 (PLK1) inhibitor, onvansertib, effectively inhibits both adrenergic and non-adrenergic contraction of smooth muscle such as human prostate tissue. This is surprising because other PLK1 inhibitors inhibit adrenergic but not non-adrenergic contraction of smooth muscle (Hennenberg et al., 2018). Because onvansertib inhibits non-adrenergic contraction of smooth muscle, specifically endothelin-1 induced, and ATP (purinergic) induced smooth muscle contractions (see Example below), onvansertib can be effectively combined with an inhibitor of adrenergic smooth muscle contraction, e.g., an α₁-blocker, to treat benign prostatic hyperplasia (BPH).

Thus, in some embodiments, a method of treating benign prostatic hyperplasia (BPH) in a patient is provided. The method comprises treating the patient with (a) onvansertib and (b) an α1-blocker.

Because onvansertib inhibits smooth muscle contraction by a different mechanism than α1-blockers. the combination treatment would be better than any of the individual inhibitors alone. In fact, the combination treatment of onvansertib and either tamsulosin or silodosin (both α1-blockers) inhibited prostate smooth muscle tissue to a greater extent than any of the individual compounds alone (see Example below).

In some embodiments, the method reduces symptoms of BPH or improves urinary flow (Qₘₐₓ) by greater than 30%. In other embodiments, the method improves urinary flow (Qₘₐₓ) by greater than 50%.

In various embodiments, the patient is treated with onvansertib and an α₁-blocker. Nonlimiting examples of α₁-blockers are silodosin, tamsulosin, alfuzosin, doxazosin, prazosin and terazosin.

Also provided is a method of inhibiting non-adrenergic contraction of a smooth muscle. The method comprises treating the smooth muscle with onvansertib.

This method can be used to inhibit any non-adrenergic smooth muscle contractions. In some embodiments, the smooth muscle contraction is endothelin-1 induced or ATP induced (purinergic).

In various embodiments of this method, the smooth muscle is prostate tissue, e.g., human prostate tissue, although this method should be effective in inhibiting non-adrenergic smooth muscle contractions in any tissue, in any mammal. In some embodiments, the prostate tissue is in a patient with benign prostatic hyperplasia (BPH).

Additionally provided is a method of inhibiting proliferation of prostate cells. The method comprises treating the prostate cells with onvansertib in a manner sufficient to inhibit growth of the human prostate cells. In various embodiments, the prostate cells are in a human patient with BPH, although this method should be effective in inhibiting prostate cell proliferation *in vitro,* or *in vivo* in any mammal.

In some embodiments of these methods, the cells are also exposed to a second pharmaceutical that inhibits proliferation of human prostate cells. The second pharmaceutical can be any pharmaceutical known to be at least partially effective in inhibiting growth of human prostate cells. A nonlimiting example of such second pharmaceutical is metformin (Wang et al., 2017).

In any of the methods provided herewith, the onvansertib may be administered by any means known in the art. In some embodiments, the onvansertib is administered orally, in a pharmaceutically acceptable excipient. Determination of the dosage of onvansertib that is effective in inhibiting smooth muscle tissue contraction or prostate cell proliferation for any specific patient can be made without undue experimentation by methods known in the art. In some embodiments, the dosage of onvansertib that is administered is greater than 6, 12, or 24 mg/m²/day. In some of these embodiments, the dosage of onvansertib is less than 50 mg/m²/day, e.g., 24 or 36 mg/m²/day.

In embodiments where the onvansertib is administered with an additional pharmaceutical, the onvansertib can be administered before, at the same time, or after the additional pharmaceutical. Additionally, the onvansertib and the second pharmaceutical can be administered on a different or the same schedule, e.g., daily, 5 days on + 2 days off, etc.

Preferred embodiments are described in the following example. Other embodiments within the scope of the claims herein will be apparent to one skilled in the art from consideration of the specification or practice of the invention as disclosed herein. It is intended that the specification, together with the examples, be considered exemplary only, with the scope of the invention being indicated by the claims, which follow the examples.

### Example. Effects of Onvansertib on Prostate Smooth Muscle Function in Human benign Prostatic Hyperplasia

### Example Abstract

Prostate smooth muscle contraction and prostate enlargement contribute to lower urinary tract symptoms suggestive of benign prostatic hyperplasia. Recent evidence demonstrated that inhibitors for polo-like kinases (PLKs) inhibit smooth muscle contraction of human prostate tissues. However, their additive effects to α₁-blockers, and effects on prostate growth are unknown. Here, we examined effects of a novel and highly selective PLK1 inhibitor, onvansertib on prostate smooth muscle contraction alone and in combination with α₁-blockers, and on proliferation and viability of prostate stromal cells (WPMY-1). Prostate tissues were obtained from radical prostatectomy. Contractions were studied in an organ bath. Proliferation and viability were assessed by plate colony, EdU, and CCK-8 assay. Electric field stimulation (EFS)-induced contractions of human prostate tissues were inhibited to 34% by 100 nM and 1 µM onvansertib at 32 Hz, and to 48% and 47% by the α₁-blockers tamsulosin and silodosin. Combination of onvansertib with tamsulosin or silodosin further reduced EFS-induced contractions in comparison to α₁-blockers alone (59% and 61% respectively), and to onvansertib alone (68% for both). Noradrenaline-, phenylephrine-, methoxamine-, endothelin-1-, and ATP-induced contractions were inhibited by onvansertib (100 nM) to similar extent. Viability and proliferation of WPMY-1 cells were reduced in a concentration and time-dependent manner (24-72 h, 10-100 nM). Onvansertib inhibits neurogenic, adrenergic, and endothelin-1- and ATP-induced contractions of human prostate smooth muscle, and proliferation of stromal cells. Contractions are reduced not more than 50% by α₁-blockers. Combination of α₁-blockers with onvansertib provides additive inhibition of prostate contractions.

### Materials and Methods

### Human prostate tissues

Human prostate tissues were obtained from patients undergoing radical prostatectomy for prostate cancer (n = 107). Patients who underwent previous transurethral resection of the prostate (TURP) were excluded. This study was carried out in accordance with the Declaration of Helsinki of the World Medical Association, and has been approved by the ethics committee of the Ludwig-Maximilians University, Munich, Germany (Ethikkommission bei der LMU München, approval number 19-737). Informed consent was obtained from all patients. According to the ethical approval, samples and data were collected and analyzed anonymously, so that no data on patients' characteristics were stored. Samples were taken immediately after prostatectomy, following macroscopical examination by an uro-pathologist. All tissues were taken from the periurethral zone, considering that most prostate cancers arise in the peripheral zone (Pradidarcheep et al., 2011; Shaikhibrahim et al., 2012). Upon pathologic evaluation, only tissue samples which did not exhibit histological signs of neoplasia, cancer, or inflammation were collected. BPH is present in 80-83% of patients with prostate cancer (Alcaraz et al., 2009; Orsted and Bojesen, 2013). Typically, such tissues show varying content of prostate-specific antigen (PSA), and different contents of smooth muscle and glandular epithelia, reflecting that BPH is present to different degree (Wang et al., 2016a, 2016c). For macroscopic examination and sampling, the prostate was opened by a single longitudinal cut from the capsule to the urethra. Subsequently, both intersections were checked macroscopically for any obvious tumor infiltration. Because tumors are usually located to the peripheral zone, tumor infiltration in the periurethral zone (where sampling was performed) was very rare (found in less than 1% of prostates). Prostates showing tumors in the periurethral zone in macroscopic inspection were not subjected to sampling and were not included in this study. Organ bath studies were performed immediately after sampling, while samples for molecular analyses were shock frozen in liquid nitrogen and stored at -80 °C.

### Tension measurements

Prostate strips (6 x 3 × 3 mm) were mounted in 10 ml aerated (95% O₂ and 5% CO₂) tissue baths (Danish Myotechnology, Aarhus, Denmark), containing Krebs-Henseleit solution (37 °C, pH 7.4) with following composition: 118 mM NaCl, 4.7 mM KCl, 2.55 mM CaCl₂, 1.2 mM KH₂PO₄, 1.2 mM MgSO₄, 25 mM NaHCO₃, 7.5 mM glucose. In each single experiment, two from four strips obtained from the same prostate were allocated to the control group (without inhibitor or antagonist), and two others to the inhibitor/antagonist group (resulting in duplicate determination for each group in each single experiment). All four samples of one experiment were examined in four chambers of the same organ bath. Consequently, control and inhibitor curves in each series/diagram were obtained from the same prostates, but different prostates were examined for different series/diagrams. The amount of solvent differed between series, due to divergent inhibitor concentrations (100 nM, 1 µM) and combination of onvansertib with α₁-blockers, what precludes any comparison between contraction levels in different series. Therefore, and considering that prostate tissues from radical prostatectomy may show considerable heterogeneity, statistical comparisons were only performed between groups within the same series (i. e. containing tissues from the same prostates for inhibitor and control group), but not between series obtained from different prostates (i. e., not across different series of organ bath experiments). Only one curve was recorded with each sample, i. e. for one agonist, or for electric field stimulation (EFS).

After mounting in organ bath chambers, preparations were stretched to 4.9 mN and left to equilibrate for 45 min. In the initial phase of the equilibration period, spontaneous decreases in tone are usually observed. Therefore, tension was adjusted three times during the equilibration period, until a stable resting tone of 4.9 mN was attained. After the equilibration period, maximum contraction induced by 80 mM KCl was assessed. Subsequently, chambers were washed three times with Krebs-Henseleit solution for a total of 30 min. Cumulative concentration response curves for noradrenaline, phenylephrine, methoxamine, endothelin-1, and for U46619, or frequency response curves induced by EFS were created 30 min after addition of onvansertib, tamsulosin, silodosin, and/or dimethylsulfoxide (DMSO) for controls. The reversibility of onvansertib effects was assessed in separate series, where DMSO and onvansertib were washed out by replacing the Krebs-Henselit solution three times within 30 min, starting 30 min after application of DMSO or onvansertib, what was followed by construction of concentration response curves for noradrenaline or of frequency response curves by EFS.

Application of EFS simulates action potentials, resulting in the release of endogenous neurotransmitters, including noradrenaline. Using the inhibitor for neurotransmitter release, tetrodotoxin, it has been previously demonstrated, that this accounts for two-thirds of EFS-induced contraction in the human prostate (Angulo et al., 2012). For EFS, tissue strips were placed between two parallel platinum electrodes connected to a CS4 stimulator (Danish Myotechnology). Square pulses with durations of 1 ms were applied with a voltage of 50 V, for a train duration of 10 s and using a delay of 1 ms between single pulses. EFS-induced contractile responses were studied at frequencies of 2, 4, 8, 16, and 32 Hz, with train intervals of 30 s between stimulations.

Data analyses and calculations were based on maximum peak heights of contractions. For calculation of agonist- or EFS-induced contractions, tensions were expressed as % of KCl-induced contractions, as this may correct different stromal/epithelial ratios, different smooth muscle content, varying degree of BPH, or any other heterogeneity between prostate samples and patients.

### Western blot analysis

Frozen prostate tissues were homogenized in a buffer containing 25 mM Tris/HCl, 10 µM phenylmethanesulfonyl fluoride, 1 mM benzamidine, and 10 µg/ml leupeptine hemisulfate, using the FastPrep^{®}-24 system with matrix A (MP Biomedicals, Illkirch, France). After centrifugation (20,000 g, 4 min), supernatants were assayed for protein concentration using the Dc-Assay kit (Biorad, Munich, Germany) and boiled for 10 min with sodium dodecyl sulfate (SDS) sample buffer (Roth, Karlsruhe, Germany). Samples of cultured stromal cells were prepared as described below. Samples (20 µg/lane) were subjected to SDS-polyacrylamide gel electrophoresis, and proteins were blotted on Protran^{®} nitrocellulose membranes (Schleicher & Schuell, Dassel, Germany). Membranes were blocked with phosphate-buffered saline (PBS) containing 5% milk powder (Roth, Karlsruhe, Germany) over night, and incubated with rabbit anti PLK1 (208G4) (#4513) (Cell Signaling Technology, Danvers, MA, USA), and mouse monoclonal anti β-actin antibody (sc-47778) (Santa Cruz Biotechnology, Santa Cruz, CA, USA).

Primary antibodies were diluted in PBS containing 0.1% Tween 20 (PBS-T) and 5% milk powder. Subsequently, detection was continued using secondary biotinylated goat anti rabbit or horse anti mouse IgG (BA-1000, BA-2000) (Vector Laboratories, Burlingame, CA, USA), followed by incubation with avidin and biotinylated horseradish peroxidase (HRP) from the "Vectastain ABC kit" (Vector Laboratories, Burlingame, CA, USA) both diluted 1:200 in PBS. Membranes were washed with PBS-T after any incubation with primary or secondary antibodies, or biotin-HRP. Finally, blots were developed with enhanced chemiluminescence (ECL) using ECL Hyperfilm (GE Healthcare, Freiburg, Germany). Intensities of resulting bands for PLK1 were quantified densitometrically using "Image J" (National Institutes of Health, Bethesda, Maryland, USA).

### RT-PCR

RNA from frozen prostate tissues or cells was isolated using the RNeasy Mini kit (Qiagen, Hilden, Germany). For isolation from tissues, 30 mg of tissue were homogenized using the FastPrep^{®}-24 system with matrix A (MP Biomedicals, Illkirch, France). RNA concentrations were measured spectrophotometrically. Reverse transcription to cDNA was performed with 1 µg of isolated RNA using the Reverse Transcription System (Promega, Madison, WI, USA). RT-PCR for PLK1, PSA, and glyceraldehyde 3-phosphate dehydrogenase (GAPDH) was performed with a Roche Light Cycler (Roche, Basel, Switzerland) using primers provided by Qiagen (Hilden, Germany) as ready-to-use mixes, based on the Ref-Seq accession numbers NM_005030 for PLK1, NM_001030047 for KLK3 (synonymous PSA), and NM_002046 for GAPDH. PCR reactions were performed in a volume of 25 µl containing 5 µl LightCycler^{®} FastStart DNA MasterPlus SYBR Green I (Roche, Basel, Switzerland), 1 µl template, 1 µl primer, and 18 µl water. Denaturation was performed for 10 min at 95 °C, and amplification with 45 cycles of 15 sec at 95 °C followed by 60 sec at 60 °C. The specificity of primers and amplification was demonstrated by subsequent analysis of melting points, which revealed single peaks for each target. Results were expressed using the ΔΔCP method, where number of cycles (Ct) at which the fluorescence signal exceeded a defined threshold for GAPDH was subtracted from Ct values for PLK (Ct_{target}-Ct_{GAPDH} = ΔCP), and values were calculated as2^{-ΔCP} and normalized to each other or to the mean values of prostate tissues.

### Cell culture

WPMY-1 cells are an immortalized cell line obtained from human prostate stroma without prostate cancer (Webber et al., 1999). Cells were obtained from American Type Culture Collection (ATCC; Manassas, VA, USA), and kept in RPMI 1640 (Gibco, Carlsbad, CA, USA) supplemented with 10% fetal calf serum (FCS) and 1% penicillin/streptomycin at 37 °C with 5% CO2. Before addition of onvansertib or DMSO, the medium was changed to a FCS-free medium. For Western blot analysis, cells were lyzed using radioimmunoprecipitation assay (RIPA) buffer (Sigma-Aldrich, St. Louis, MO, USA), and removed from flasks after 15 min of incubation on ice. Cell debris was removed by centrifugation (10,000 g, 10 min, 4 °C), and different aliquots of supernatants were either subjected to protein determination, or boiled with SDS sample buffer.

### Cytotoxicity assay

Viability of cells was assessed using the Cell Counting Kit-8 (CCK-8) (Sigma-Aldrich, St. Louis, MO, USA). Cells were grown in 96-well plates (5000 cells/well) for 24 h, before onvansertib or solvent were added in indicated concentrations. Subsequently, cells were grown for different periods (24, 48, 72 h). Separate controls were performed for each period. At the end of this period, 10 µl of [2-(2-methoxy-4-nitrophenyl)-3-(4-nitrophenyl)-5-(2,4-disulfophenyl)-2H-tetrazolium monosodium salt (WST-8) from CCK-8 was added, and absorbance in each well was measured at 450 nm after incubation for 2 h at 37 °C.

### Colony formation assay

About 100 cells were placed to each well of a 6-well culture plate, and were treated with onvansertib (final concentrations 10, 50, 100 nM) or solvent. Cells were incubated at 37 °C for 14 days, then washed twice with PBS, and fixed by 2 ml of 10% trichloroacetic acid overnight (4 °C). Subsequently, all plates were washed five times with cold water, and stained with 0.4% sulforhodamine B solution (diluted in 1% acetic acid) at room temperature for 30 min. Before taking photos, all plates were labeled and washed five times with 1% acetic acid. The number of colonies containing 50 cells or more was counted under a microscope.

### EdU proliferation assay

WPMY-1 cells were plated with cell line-specific densities (50,000/well) on 16-well chambered coverslips (Thermo Scientific, Waltham, MA, USA). After 24 h, cells were treated with onvansertib (final concentration 10, 50, 100 nM) or solvent. After further 24 h, the medium was changed to a 10 mM 5-ethynyl-2'-deoxyuridine (EdU) solution in FCS-free medium containing onvansertib or solvent. 20 h later, cells were fixed with 3.7% formaldehyde. EdU incorporation was determined using the "EdU-Click 555" cell proliferation assay (Baseclick, Tutzing, Germany) according to the manufacturer's instructions. In this assay, incorporation of EdU into DNA is assessed by detection with fluorescing 5-carboxytetramethylrhodamine (5-TAMRA). Counterstaining of all nuclei was performed with DAPI. Cells were analyzed by fluorescence microscopy (excitation: 546 nm; emission: 479 nm).

### Drugs and nomenclature

Onvansertib (PCM-075) (1-(2-hydroxyethyl)-8-[5-(4-methylpiperazin-1-yl)-2-(trifluoromethoxy)anilino]-4,5-dihydropyrazolo[4,3-h]quinazoline-3-carboxamide) is a PLK inhibitor with high selectivity for PLK1 (Beria et al., 2011). Phenylephrine ((R)-3-[-1-hydroxy-2-(methylamino)ethyl]phenol) and methoxamine (α-(1-Aminoethyl)-2,5-dimethoxybenzyl alcohol) are selective agonists for α₁-adrenoceptors. U46619 ((Z)-7-[(1S,4R,5R, 6S)-5-[(E, 3S)-3-hydroxyoct-1-enyl]-3-oxabicyclo [2.2.1]heptan-6-yl]hept-5-enoic acid) is an analog of thromboxane A2 (TXA2), which specifically activates thromboxane A2 receptors, and is frequently used as an agonist for thromboxane A2 receptors (Malmsten, 1976; Shen and Tai, 1998). Tamsulosin (5-[(2 R)-2-[2-(2-ethoxyphenoxy)ethylamino]propyl]-2-methoxybenzenesulfonamide) and silodosin (1-(3-hydroxypropyl)-5-[(2 R)-2-[2-[2-(2,2,2-trifluoroethoxy)phenoxy]ethylamino]propyl]-2,3-dihydroindole-7-carboxamide) are α₁-adrenoceptor antagonists, with high selectivity for the α_{1A} subtype. Both are available and routinely used for treatment of LUTS suggestive of BPH (Oelke et al., 2013). Stock solutions of onvansertib (10 mM) and appropriate dilutions were prepared in DMSO, and stored at -20 °C until use. Aqueous stock solutions of noradrenaline, phenylephrine, methoxamine (all 10 mM), and adenosine 5'-triphosphate (ATP) (500 mM suspension) were freshly prepared before each experiment. Stock solutions of U46619 (10 mM) were prepared in ethanol, and stored at -80 °C until use. Aqueous stock solutions of endothelin-1 (0.4 mM) were stored at -20 °C until used. Stock solutions of tamsulosin and silodosin (10 mM) were prepared in DMSO, and further diluted (1:100, to 100 µM) with distilled water and stored at -20 °C until use. Onvansertib was provided from Trovagene Inc. (San Diego, CA, USA). Noradrenaline, phenylephrine, methoxamine, and ATP were obtained from Sigma (Munich, Germany), U46619, tamsulosin, and silodosin from Tocris (Bristol, UK), and endothelin-1 from Enzo Life Sciences (Lörrach, Germany).

### Statistical analysis

Data are presented as means ± standard error of the mean (S.E.M.) with the indicated number (n) of experiments. Multivariate analysis of variance (ANOVA) and two-way ANOVA were used for unpaired observations, and performed using SPSS^{®} version 20 (IBM SPSS Statistics, IBM Corporation, Armonk, New York, USA). P values < 0.05 were considered statistically significant. All groups included in the statistical analyses were based on five or more independent experiments, and included tissues from five or more patients in each group in the case of experiments performed with human tissues. Thus, the minimum group size subjected to statistical tests was n = 5. Moreover, all groups being compared with each other by statistical tests showed identical group sizes; consequently, any statistical comparison between groups of different sample sizes, or between groups composed with tissues from different patients were not performed. Spearman's correlation analysis and calculation of pEC50 values by curve fitting were performed using GraphPadPrism 6 (Statcon, Witzenhausen, Germany).

### Results

### Effects of onvansertib on adrenergic contractions

Contractions of human prostate tissues were induced in an organ bath, to assess effects of onvansertib and α₁-blockers on prostate smooth muscle contraction. Effects of onvansertib on noradrenaline-induced contractions of human prostate tissues were tested using two different concentrations of onvansertib. Both concentrations, i. e. 100 nM and 1 µM caused significant inhibitions of noradrenaline-induced contractions (FIG. 1A). The extent of inhibition was similar for both concentrations, so that increasing of the onvansertib concentration from 100 nM to 1 µM did not increase the degree of inhibition (45 ± 9.7% inhibition of 100 µM noradrenaline-induced contractions by 100 nM onvansertib, 41 ± 6.2% inhibition of 100 µM noradrenaline-induced contractions by 1 µM onvansertib). In contrast to the contractile forces, onvansertib did not change the pEC50 values for noradrenaline (5.9 ± 0.2 M following 100 nM onvansertib and 5.5 ± 0.2 M in corresponding controls; 5.7 ± 0.3 M following 1 µM onvansertib and 6 ± 0.3 M in corresponding controls).

Effects of onvansertib on α₁-adrenergic prostate smooth muscle contractions were confirmed using the two α₁-adrenoceptor agonists, phenylephrine and methoxamine. Onvansertib (100 nM) caused significant inhibitions of phenylephrine- and methoxamine-induced contractions (FIG. 1B). The extent of inhibition was similar for both agonists, and resembled the inhibition of noradrenaline-induced contractions by onvansertib (47 ± 16.2% inhibition of 30 µM phenylephrine-induced contractions by 100 nM onvansertib, 43 ± 19.4% inhibition of 100 µM methoxamine-induced contractions by 100 nM onvansertib). In contrast to the contractile forces, onvansertib did not change the pEC₅₀ values for phenylephrine or methoxamine (7.8 ± 1.3 M for phenylephrine following 100 nM onvansertib and 6.7 ± 0.8 M in corresponding controls; 5.2 ± 0.2 for methoxamine following 100 nM onvansertib and 5 ± 0.1 in corresponding controls).

To assess the reversibility of onvansertib effects on adrenergic contractions, noradrenaline-induced contractions were assessed following washout of DMSO and onvansertib (100 nM) in a separate series of experiments. Under these conditions, noradrenaline-induced contractions did not differ, i. e. were similar after washout of DMSO and of onvansertib (FIG. 1C).

### Effects of onvansertib on non-adrenergic contractions

Effects of onvansertib were tested on prostate smooth muscle contractions induced by endothelin-1, ATP, or the thromboxane A2 analog U46619. Onvansertib (100 nM) caused significant inhibition of endothelin-1-induced contractions (45 ± 6.6% inhibition of 3 µM endothelin-1-induced contraction by 100 nM onvansertib), and of ATP-induced contractions (51 ± 18.6% inhibition of 10 mM ATP-induced contraction by 100 nM onvansertib) (FIG. 2). Onvansertib (100 nM) did not change U46619-induced contractions (FIG. 2).

### Effects of onvansertib on EFS-induced contractions

Effects of onvansertib on neurogenic contractions of human prostate tissues induced by EFS were tested using two different concentrations of onvansertib. Both concentrations, i. e. 100 nM and 1 µM caused significant inhibitions of EFS-induced contractions (FIG. 3A). The extent of inhibition was similar for both concentrations, so that increasing of the onvansertib concentration from 100 nM to 1 µM did not increase the extent of inhibition (34 ± 11.3% inhibition of 32 Hz-induced contractions by 100 nM onvansertib, 34 ± 14% inhibition of 32 Hz-induced contractions by 1 µM onvansertib).

To assess the reversibility of onvansertib effects on EFS-induced contractions, EFS-induced contractions were assessed following washout of DMSO and onvansertib (100 nM) in a separate series of experiments. Under these conditions, EFS-induced contractions did not differ, i.e. were similar after washout of DMSO and of onvansertib (FIG. 3B).

### Effects of α₁-blockers on EFS- and noradrenaline-induced contractions

The α₁-blockers tamsulosin and silodosin inhibited EFS-induced contractions of human prostate tissues significantly and to similar degree (FIG. 4A). The extent of inhibition was similar for both compounds (48 ± 18.3% inhibition of 32 Hz-induced contractions by 300 nM tamsulosin, 47 ± 16.4% inhibition of 32 Hz-induced contractions by 100 nM silodosin). In contrast to the incomplete inhibition of EFS-induced contractions, the same concentrations of tamsulosin and silodosin inhibited noradrenaline-induced contractions nearly completely (93 ± 3.5% inhibition of 100 µM noradrenaline-induced contractions by 300 nM tamsulosin, 84 ± 4.99% inhibition of 100 µM noradrenaline-induced contractions by 100 nM silodosin) (FIG. 4B).

### Effects of combination of onvansertib with α₁-blockers on EFS-induced contractions

EFS-induced contractions were compared between human prostate tissues after addition of α1-blocker alone or of a combination of α₁-blocker and 100 nM onvansertib (FIG. 5a). EFS-induced contractions were significantly lower after addition of the combination of α₁-blocker with onvansertib compared to EFS-induced contractions after addition of α₁-blocker alone (FIG. 5a). The effect of the add-on of onvansertib was similar using tamsulosin and silodosin (59 ± 11.5% inhibition of 32 Hz-induced contraction by onvansertib compared to tamsulosin alone, 61 ± 9.8% inhibition of 32 Hz-induced contraction by onvansertib compared to silodosin alone).

In two separate series of experiments, effects of combinations of onvansertib and α₁-blocker on EFS-induced contractions were compared to effects of onvansertib (100 nM) alone (FIG. 5b). EFS-induced contractions were significantly lower after addition of the combination of onvansertib with an α₁-blocker compared to EFS-induced contractions after addition of onvansertib alone (FIG. 5b). The effect of the add-on of α₁-blocker was similar using tamsulosin and silodosin (68 ± 12.2% inhibition of 32 Hz-induced contraction by tamsulosin compared to onvansertib alone, 68 ± 12.7% inhibition of 32 Hz-induced contraction by silodosin compared to onvansertib alone).

### Detection of PLK1 in WPMY-1 cells

To compare possible expression levels of PLK1 in the human prostate stromal cell line, WPMY-1, with human prostate tissues, Western blot analysis was performed using an antibody raised for PLK1. Detection revealed bands with the size of the expected molecular weight for PLK1 (FIG. 6). In line with previous findings (Hennenberg et al., 2018), the intensity of these bands varied highly between samples of prostate tissues from different patients (FIG. 6A). In contrast, the intensity was constant for four different samples of WPMY-1 cells, and ranged at the highest expression levels observed for human prostate tissues (FIG. 6B). Similar patterns were observed after detection of PLK1 mRNA by RT-PCR (FIG. 6C). Thus, the content of PLK1 mRNA showed high variations in human prostate tissues (FIG. 6C). In WPMY-1 cells, the content of PLK1 mRNA was constant and ranged at a level similar to the highest contents observed in prostate tissues (FIG. 6C). In human prostate tissues, no correlation was observed between mRNA levels of PLK1 and PSA (R = -0.543, P = 0.297) (FIG. 6D).

### Effects of onvansertib on viability and proliferation of WPMY-1 cells

Effects of onvansertib on viability of WPMY-1 cells were assessed by CCK-8 assay. Onvansertib induced concentration- (10 nM, 50 nM, 100 nM) and time- (24-72 h) dependent decreases of viability (FIG. 7A). Decreases ranged between 15 ± 8.8% from 10 nM to 63 ± 2.2% from 100 nM after 24 h, 28 ± 8.3% from 10 nM to 86 ± 1.9% from 100 nM after 48 h, and 40 ± 4.4% from 10 nM to 89 ± 1.4% from 100 nM after 72 h (FIG. 7A).

Effects of onvansertib on proliferation of WPMY-1 cells were assessed by two different readouts, with results confirming each other (FIG. 7B and C). In a plate colony assay, colony formation was reduced concentration-dependently by onvansertib, amounting to decreases of 39 ± 4.5% by 10 nM, 89 ± 1.9% by 50 nM, and 97 ± 0.4% by 100 Nm (FIG. 7B). In an EdU assay, the percentage of proliferating cells was reduced concentration-dependently by onvansertib, amounting to decreases of 18 ± 0.1% by 10 nM, 24 ± 2.1% by 50 nM, and 35 ± 2.0% by 100 nM after 24 h (FIG. 7C).

### Discussion

Our findings suggest that the PLK1 inhibitor onvansertib inhibits 1) neurogenic, α1-adrenergic, and endothelin-induced contractions in the human prostate, and 2) proliferation of prostate stromal cells. Notably, combination of onvansertib with α1-blockers caused larger inhibitions of neurogenic contractions than α1-blockers alone. The additive effects of onvansertib and α1-blockers, together with the simultaneous effect on growth may be of particular interest from a clinical point of view. Prostate smooth muscle contraction and prostate growth may both contribute to BOO and LUTS in BPH (Hennenberg et al., 2014). Consequently, both are targets for medical therapy, which are, however, still afflicted by insufficient effectiveness, common use of combination therapies, and low adherence, finally resulting in complications, hospitalization, and surgery (Hennenberg et al., 2014; Oelke et al., 2013). Identification of novel targets is a prerequisite to overcome these limitations in the future. Based on our findings, onvansertib may be an attractive compound to be tested in the context of LUTS suggestive of BPH in vivo.

We tested the effects of onvansertib on EFS- and noradrenaline-induced contractions using two different concentrations. Both concentrations, 100 nM and 1 µM of onvansertib inhibited contractions to similar extent. Therefore, together with reported IC50 values, we conclude that the inhibition of contraction was largely dependent on PLK1-inhibition, and that no other contraction-mediating kinases were involved in this range up to 1 µM. In fact, onvansertib has been reported to be highly PLK1-specific, showing IC50 values of 2 nM for PLK1, 10 µM for PLK2, and 10 µM for PLK3 in biochemical assays (Beria et al., 2011). In another screen by biochemical assays, onvansertib was tested against 296 kinases, and showed decreases in activities (<50%) of 11 kinases (including PLK1) when tested at 1 µM, and inhibition only of PLK1 when tested at 100 nM (Valsasina et al., 2012). Another series of biochemical assays in the same study performed with 63 kinases revealed IC₅₀ values of 510 nM for FLT3, 744 nM for MELK, and 826 nM for CK2 (Valsasina et al., 2012). Therefore, the concentration of 100 nM used in most of our experiments may be highly specific for PLK1, and most parts of the effects may be attributed to PLK1 inhibition. As the concentration of 1 µM did not provide larger inhibition than the inhibition resulting from 100 nM, we assume 1) that 1 µM does not cause off-target inhibition of contraction-mediating kinases, and 2) that 100 nM is sufficient to attain the maximum, PLK1-dependent effect. In line with previous studies reporting that onvansertib binds to the ATP pocket of PLK1 by hydrogen bonds and consequently acts as an ATP competitive inhibitor with reversible dissociation (Beria et al., 2011; Valsasina et al., 2012), the effects of onvansertib in our study turned out to be reversible. Thus, following washout of onvansertib, contractions recovered to levels of corresponding controls.

Inhibition of smooth muscle contractions in our organ bath experiments was observed, although the history of included patients was not taken into account. Thus, tissue samples were anonymized, and no patients data were collected, stored, or analyzed for this study. To eliminate heterogeneities resulting from individual variations or from previous treatment, we referred contractions to highmolar KCl-induced contractions, and confirmed our findings by different settings, i. e. using different α1-adrenoceptor agonists or different combination settings. Nevertheless, lacking reference to patients' characteristics, in particular to previous medications which may influence contractility, needs to be considered as a potential limitation. The inhibition of neurogenic and α1-adrenergic prostate smooth muscle contractions is in line with recent findings obtained with four other inhibitors with assumed selectivity for PLK1 (Hennenberg et al., 2018). Similar to our current results, the inhibition of EFS-induced contractions by other PLK1 inhibitors ranged around 50% (Hennenberg et al., 2018). Here, we tested effects of two α₁-blockers on EFS-induced contractions, and compared combinations of onvansertib with α₁-blockers to α₁-blockers and onvansertib alone. Both α₁-blockers, tamsulosin and silodosin provided very similar results, and inhibited EFS-induced contractions less than 50%. This incomplete inhibiton by α₁-blockers is in line with previous results, reporting similar or even smaller effects of α₁-blockers in EFS-induced contractions of human prostate tissues (Angulo et al., 2012; Buono et al., 2014; Chueh et al., 1996; Oger et al., 2009, 2010). We exclude, that the incomplete inhibition of EFS-induced contractions by tamsulosin and silodosin resulted from too low concentrations of α₁-blockers. In fact, the same concentrations used in EFS experiments reduced noradrenaline-induced prostate contractions completely (at least in the range of noradrenaline concentrations applied here). We observed that EFS-induced contractions were significantly lower after application of a combination of onvansertib and α₁-blocker compared to tensions after α₁-blocker or onvansertib alone, what may be considered as a key finding of our study. It may be speculated, that add-on of onvansertib or other PLK1 inhibitors to α₁-blockers may result in higher improvements of LUTS than α₁-blockers alone in patients with BPH. In line with previous results obtained at protein level, we here confirmed that PLK1 mRNA expression does not correlate with PSA expression, suggesting that it acts independently from the degree of BPH (Hennenberg et al., 2018).

In contrast to recent findings, where the PLK1 inhibitors SBE 13 and cyclapolin did not change endothelin-1-induced contractions of human prostate tissues, we here observed an inhibition of endothelin-1-induced contractions by onvansertib (Hennenberg et al., 2018). The reason for this discrepancy remains speculation at this stage, but may reflect divergent pharmacologic profiles. However, it is notable thateven the inhibition of adrenergic contractions (induced by noradrenaline, phenylephrine, and methoxamine) appears larger for onvansertib, than the inhibition reported for SBE 13 and cyclapolin 9 (Hennenberg et al., 2018). In addition to endothelin-1-induced contractions, onvansertib inhibited contractions induced by ATP. Notably, our data may be the first evidence suggesting purinergic smooth muscle contractions in the human prostate, as previous investigations addressing ATP-induced contractions in the prostate were to the best of our knowledge limited to animal models (Brandli et al., 2010; Buljubasich and Ventura, 2004; Hennenberg et al., 2017; White et al., 2015; Xu and Ventura, 2010). In line with other PLK inhibitors, U46619-induced contractions were here again resistant to onvansertib. Nonadrenergic mediators including endothelin-1, thromboxane A2, and probably ATP may contribute to prostate smooth muscle tone in parallel to α1-adrenoceptors in BPH, so that they could maintain urethral obstruction despite treatment with α1-blockers, what may explain the restrictions of α1-blockers or the high number of non-responders (Hennenberg et al., 2014, 2017). Considering the effect on endothelin-1- and ATP-induced contractions, improvements of LUTS suggestive of BPH by onvansertib in vivo may exceed those of α₁-blockers.

As another key finding, we observed that onvansertib inhibited the proliferation of prostate stromal cells. Previous studies reporting inhibition of proliferation by onvansertib were performed in different types of tumor cells (Casolaro et al., 2013; Hartsink-Segers et al., 2013; Sero et al., 2014; Valsasina et al., 2012). In the prostate, other PLK inhibitors or knockdown of PLK1 inhibit the proliferation of tumor cells and tumor growth, and enhance the effectiveness of antitumor treatments (Lin et al., 2019; Reagan-Shaw and Ahmad, 2005; Shao et al., 2015; Zhang et al., 2014). Generally, PLK inhibitors including onvansertib reduce proliferation by a G2-M cell cycle block (Valsasina et al., 2012). A prolonged mitotic block by PLK inhibition can be followed by apoptosis and cell death (Valsasina et al., 2012). Both processes may account for the effects of onvansertib on proliferation and viability of WPMY-1 cells in our study. Reduced viability in WPMY-1 cells occurred as early as 24 h, but was obviously time-dependent. Regarding smooth muscle cells, a role of PLK1 for promoting proliferation has been suggested for airway smooth muscle cells, where PLK1 knockdown inhibited growth factor-induced proliferation (Jiang and Tang, 2015). Together, our observation that proliferation and viability of WPMY-1 cells are affected by a PLK1 inhibitor is in line with previous studies, which were performed in other cell types. WPMY-1 cells are derived from non-malignant human prostate stroma, and strongly resemble prostate smooth muscle cells, and may be considered as such (Wang et al., 2015). Smooth muscle cells are the predominant cell type in the prostate stroma, which may show increased proliferation in BPH (Strand et al., 2017). The latter contributes to prostate enlargement in BPH (Strand et al., 2017). Comparison of expression levels by Western blot and RT-PCR suggested, that WPMY-1 cells express PLK1 at a constant level, which is similar to highest levels in human prostate tissues, where the PLK1 content varies. It may be expected that onvansertib could prevent prostate growth or reduce prostate size in BPH, what is a principal aim in treatment with 5-ARIs, in order to prevent progression of BPH and to reduce the risk for complications and surgery (Oelke et al., 2013).

Prostate growth and smooth muscle contraction are the targets for medical therapy in BPH, but were mostly considered separately from each other for long time. Based on recent evidence showing that prostate smooth muscle contraction and growth of stromal cells are susceptible to the same inhibitors, it becomes now increasingly clear that both processes are closely connected with each other (Hennenberg et al., 2014). Inhibitors for Rho kinase, Rac GTPases, or Src family kinases simultaneously inhibited agonist-induced contractions of human prostate tissues and growth of prostate stromal cells (Rees et al., 2003; Wang et al., 2015, 2016b). This may resemble to the situation in other smooth muscle cells, e. g. vascular smooth muscle cells, where RhoA/Rho kinase mediate contraction and proliferation (Shimokawa et al., 2016). Notably, a similar role has been suggested for PLK1 in airway smooth muscle cells, where proliferation and contraction both depend on PLK1 (Jiang and Tang, 2015; Li et al., 2016). The same applies obviously for prostate smooth muscle. Thus, in contrast to α¹-blockers, or 5-ARIs, onvansertib may reduce prostate smooth muscle tone and growth at once. Whether onvansertib addresses the same or different intracellular signaling pathways to inhibit smooth muscle contraction and proliferation, and which other effectors take part together with PLK1 in onvansertib-sensitive signaling, can not be estimated on the basis of our data. Effects of PLK inhibitors on vascular smooth muscle contraction, which may cause cardiovascular side effects and could limit an application in vivo, have to the best of our knowledge not been reported to date.

Together, it may be speculated for three reasons that onvansertib may be highly effective in vivo, if it is applied for treatment of LUTS suggestive of BPH. First, it may be assumed that improvements of LUTS by add-on of onvansertib to α1-blockers may be higher than by α1-blockers alone, what is based on our findings from combination experiments in EFS-induced contractions. Secondly, a high degree of symptom reduction and improvement of Qₘₐₓ may be expected, because onvansertib inhibited endothelin-1-induced contractions, which are not susceptible to α₁-blockers, but contribute to prostate smooth muscle tone in BPH (Hennenberg et al., 2014, 2017). Third, a reduction of prostate size may be expected in parallel to symptom reduction, as onvansertib strongly reduced the viability of stromal cells. Following chronic application in vivo, the antiproliferative effect may reduce contractility even stronger than the short time exposure in the organ bath, as the number of smooth muscle cells may be reduced in vivo. In the organ bath, exposure for 1 h is usually not sufficient to reduce contractility by affecting viability (Yu et al., 2018, 2019a, 2019b), so that the anticontractile effect occurred most likely from a PLK1-mediated signaling mechanism, which promotes contraction. Combination therapies of α₁-blockers and 5-ARIs are commonly applied for treatment of male LUTS (Oelke et al., 2013). However, side effects are additive, and discontinuation rates are high (Fullhase et al., 2013). Single compounds such as onvansertib, which may address adrenergic and nonadrenergic prostate smooth muscle tone together with growth at once, may be attractive for clinical trials, as they may replace combination therapies of α₁-blockers with 5-ARIs and may exceed the effectiveness of monotherapy with α₁-blockers. Onvansertib is orally available (Beria et al., 2011; Valsasina et al., 2012). Pharmacokinetics and safety were recently examined in a phase I study, where plasma concentrations in a three-digit nanomolar range were reached in humans, even by the lowest dosages tested (Weiss et al., 2018). Consequently, it appears possible, that concentrations resembling those in our organ bath experiments (100 nM) may be obtained in the prostate by oral administration, so that clinical studies in the context of LUTS suggestive of BPH may be promising.

### Conclusions

Onvansertib inhibits neurogenic and α₁-adrenergic contractions of human prostate smooth muscle and proliferation of prostate stromal cells. Neurogenic contractions are reduced not more than 50% by α₁-blockers, but combination of α₁-blockers with onvansertib provides additive and stronger inhibition of neurogenic prostate contractions. It appears possible that onvansertib may reduce prostate smooth muscle tone and growth at once in BPH, so that onvansertib could be an attractive compound to be tested in vivo in the context of LUTS suggestive of BPH.

### References

Alcaraz, A., Hammerer, P., Tubaro, A., Schroder, F.H., Castro, R., 2009. Is there evidence of a relationship between benign prostatic hyperplasia and prostate cancer? Findings of a literature review. Eur. Urol. 55, 864-873.
Angulo, J., Cuevas, P., Fernandez, A., La Fuente, J.M., Allona, A., Moncada, I., Saenz de Tejada, I., 2012. Tadalafil enhances the inhibitory effects of tamsulosin on neurogenic contractions of human prostate and bladder neck. J. Sex. Med. 9, 2293-2306.
Beria, I., Bossi, R.T., Brasca, M.G., Caruso, M., Ceccarelli, W., Fachin, G., Fasolini, M., Forte, B., Fiorentini, F., Pesenti, E., Pezzetta, D., Posteri, H., Scolaro, A., Re Depaolini, S., Valsasina, B., 2011. NMS-P937, a 4,5-dihydro-1H-pyrazolo[4,3-h]quinazoline derivative as potent and selective Polo-like kinase 1 inhibitor. Bioorg. Med. Chem. Lett 21, 2969-2974.
Brandli, A., Simpson, J.S., Ventura, S., 2010. Isoflavones isolated from red clover (Trifolium pratense) inhibit smooth muscle contraction of the isolated rat prostate gland. Phytomedicine 17, 895-901.
Buljubasich, R., Ventura, S., 2004. Adenosine 5'-triphosphate and noradrenaline are excitatory cotransmitters to the fibromuscular stroma of the Guinea pig prostate gland. Eur. J. Pharmacol. 499, 335-344.
Buono, R., Briganti, A., Freschi, M., Villa, L., La Croce, G., Moschini, M., Benigni, F., Castiglione, F., Montorsi, F., Hedlund, P., 2014. Silodosin and tadalafil have synergistic inhibitory effects on nerve-mediated contractions of human and rat isolated prostates. Eur. J. Pharmacol. 744, 42-51.
Caine, M., Pfau, A., Perlberg, S., 1976. The use of alpha-adrenergic blockers in benign prostatic obstruction. Br. J. Urol. 48, 255-263.
Caine, M., Raz, S., Zeigler, M., 1975. Adrenergic and cholinergic receptors in the human prostate, prostatic capsule and bladder neck. Br. J. Urol. 47, 193-202.
Casolaro, A., Golay, J., Albanese, C., Ceruti, R., Patton, V., Cribioli, S., Pezzoni, A., Losa, M., Texido, G., Giussani, U., Marchesi, F., Amboldi, N., Valsasina, B., Bungaro, S., Cazzaniga, G., Rambaldi, A., Introna, M., Pesenti, E., Alzani, R., 2013. The Polo-Like Kinase 1 (PLK1) inhibitor NMS-P937 is effective in a new model of disseminated primary CD56+ acute monoblastic leukaemia. PloS One 8, e58424.
Chueh, S.C., Guh, J.H., Chen, J., Lai, M.K., Ko, F.N., Teng, C.M., 1996. Inhibition by tamsulosin of tension responses of human hyperplastic prostate to electrical field stimulation. Eur. J. Pharmacol. 305, 177-180.
Cindolo, L., Pirozzi, L., Fanizza, C., Romero, M., Tubaro, A., Autorino, R., De Nunzio, C., Schips, L., 2015. Drug adherence and clinical outcomes for patients under pharmacological therapy for lower urinary tract symptoms related to benign prostatic hyperplasia: population-based cohort study. Eur. Urol. 68, 418-425.
Dahm, P., Brasure, M., MacDonald, R., Olson, C.M., Nelson, V.A., Fink, H.A., Rwabasonga, B., Risk, M.C., Wilt, T.J., 2017. Comparative effectiveness of newer medications for lower urinary tract symptoms attributed to benign prostatic hyperplasia: a systematic review and meta-analysis. Eur. Urol. 71, 570-581.
de Carcer, G., Wachowicz, P., Martinez-Martinez, S., Oller, J., Mendez-Barbero, N., Escobar, B., Gonzalez-Loyola, A., Takaki, T., El Bakkali, A., Camara, J.A., Jimenez-Borreguero, L.J., Bustelo, X.R., Canamero, M., Mulero, F., de Los Angeles Sevilla, M., Montero, M.J., Redondo, J.M., Malumbres, M., 2017. Plk1 regulates contraction of postmitotic smooth muscle cells and is required for vascular homeostasis. Nat. Med..
Fullhase, C., Chapple, C., Cornu, J.N., De Nunzio, C., Gratzke, C., Kaplan, S.A., Marberger, M., Montorsi, F., Novara, G., Oelke, M., Porst, H., Roehrborn, C., Stief, C., McVary, K.T., 2013. Systematic review of combination drug therapy for non-neurogenic male lower urinary tract symptoms. Eur. Urol. 64, 228-243.
Hartsink-Segers, S.A., Exalto, C., Allen, M., Williamson, D., Clifford, S.C., Horstmann, M., Caron, H.N., Pieters, R., Den Boer, M.L., 2013. Inhibiting Polo-like kinase 1 causes growth reduction and apoptosis in pediatric acute lymphoblastic leukemia cells. Haematologica 98, 1539-1546.
Hennenberg, M., Acevedo, A., Wiemer, N., Kan, A., Tamalunas, A., Wang, Y., Yu, Q., Rutz, B., Ciotkowska, A., Herlemann, A., Strittmatter, F., Stief, C.G., Gratzke, C., 2017. Non-adrenergic, tamsulosin-insensitive smooth muscle contraction is sufficient to replace alpha 1 - adrenergic tension in the human prostate. Prostate 77, 697-707.
Hennenberg, M., Kuppermann, P., Yu, Q., Herlemann, A., Tamalunas, A., Wang, Y., Rutz, B., Ciotkowska, A., Strittmatter, F., Stief, C.G., Gratzke, C., 2018. Inhibition of prostate smooth muscle contraction by inhibitors of polo-like kinases. Front. Physiol. 9, 734.
Hennenberg, M., Stief, C.G., Gratzke, C., 2014. Prostatic alpha 1-adrenoceptors: new concepts of function, regulation, and intracellular signaling. Neurourol. Urodyn. 33, 1074-1085.
Irwin, D.E., Kopp, Z.S., Agatep, B., Milsom, I., Abrams, P., 2011. Worldwide prevalence estimates of lower urinary tract symptoms, overactive bladder, urinary incontinence and bladder outlet obstruction. BJU Int. 108, 1132-1138.
Jiang, S., Tang, D.D., 2015. Plk1 regulates MEK1/2 and proliferation in airway smooth muscle cells. Respir. Res. 16, 93.
Li, J., Wang, R., Gannon, O.J., Rezey, A.C., Jiang, S., Gerlach, B.D., Liao, G., Tang, D.D., 2016. Polo-like kinase 1 regulates vimentin phosphorylation at ser-56 and contraction in smooth muscle. J. Biol. Chem. 291, 23693-23703.
Lin, C., Bai, S., Du, T., Lai, Y., Chen, X., Peng, S., Ma, X., Wu, W., Guo, Z., Huang, H., 2019. Polo-like kinase 3 is associated with poor prognosis and regulates proliferation and metastasis in prostate cancer. Canc. Manag. Res. 11, 1517-1524.
Malmsten, C., 1976. Some biological effects of prostaglandin endoperoxide analogs. Life Sci. 18, 169-176.
Oelke, M., Bachmann, A., Descazeaud, A., Emberton, M., Gravas, S., Michel, M.C., N'Dow, J., Nordling, J., de la Rosette, J.J., European Association of, U., 2013. EAU guidelines on the treatment and follow-up of non-neurogenic male lower urinary tract symptoms including benign prostatic obstruction. Eur. Urol. 64, 118-140.
Oger, S., Behr-Roussel, D., Gorny, D., Lebret, T., Denoux, Y., Alexandre, L., Giuliano, F., 2010. Combination of alfuzosin and tadalafil exerts an additive relaxant effect on human detrusor and prostatic tissues in vitro. Eur. Urol. 57, 699-707.
Oger, S., Behr-Roussel, D., Gorny, D., Lecoz, O., Lebret, T., Denoux, Y., Faix, A., Leriche, A., Wayman, C., Alexandre, L., Giuliano, F., 2009. Combination of doxazosin and sildenafil exerts an additive relaxing effect compared with each compound alone on human cavernosal and prostatic tissue. J. Sex. Med. 6, 836-847.
Orsted, D.D., Bojesen, S.E., 2013. The link between benign prostatic hyperplasia and prostate cancer. Nat. Rev. Urol. 10, 49-54.
Pradidarcheep, W., Wallner, C., Dabhoiwala, N.F., Lamers, W.H., 2011. Anatomy and histology of the lower urinary tract. Handb. Exp. Pharmacol. 117-148.
Reagan-Shaw, S., Ahmad, N., 2005. Silencing of polo-like kinase (Plk) 1 via siRNA causes induction of apoptosis and impairment of mitosis machinery in human prostate cancer cells: implications for the treatment of prostate cancer. Faseb. J. 19, 611-613.
Rees, R.W., Foxwell, N.A., Ralph, D.J., Kell, P.D., Moncada, S., Cellek, S., 2003. Y-27632, a Rho-kinase inhibitor, inhibits proliferation and adrenergic contraction of prostatic smooth muscle cells. J. Urol. 170, 2517-2522.
Sero, V., Tavanti, E., Vella, S., Hattinger, C.M., Fanelli, M., Michelacci, F., Versteeg, R., Valsasina, B., Gudeman, B., Picci, P., Serra, M., 2014. Targeting polo-like kinase 1 by NMS-P937 in osteosarcoma cell lines inhibits tumor cell growth and partially overcomes drug resistance. Invest. N. Drugs 32, 1167-1180.
Shaikhibrahim, Z., Lindstrot, A., Ellinger, J., Rogenhofer, S., Buettner, R., Perner, S., Wernert, N., 2012. The peripheral zone of the prostate is more prone to tumor development than the transitional zone: is the ETS family the key? Mol. Med. Rep. 5, 313-316.
Shao, C., Ahmad, N., Hodges, K., Kuang, S., Ratliff, T., Liu, X., 2015. Inhibition of polo-like kinase 1 (Plk1) enhances the antineoplastic activity of metformin in prostate cancer. J. Biol. Chem. 290, 2024-2033.
Shen, R.F., Tai, H.H., 1998. Thromboxanes: synthase and receptors. J. Biomed. Sci. 5, 153-172.
Shimokawa, H., Sunamura, S., Satoh, K., 2016. RhoA/rho-kinase in the cardiovascular system. Circ. Res. 118, 352-366.
Strand, D.W., Costa, D.N., Francis, F., Ricke, W.A., Roehrborn, C.G., 2017. Targeting phenotypic heterogeneity in benign prostatic hyperplasia. Differentiation 96, 49-61.
Valsasina, B., Beria, I., Alli, C., Alzani, R., Avanzi, N., Ballinari, D., Cappella, P., Caruso, M., Casolaro, A., Ciavolella, A., Cucchi, U., De Ponti, A., Felder, E., Fiorentini, F., Galvani, A., Gianellini, L.M., Giorgini, M.L., Isacchi, A., Lansen, J., Pesenti, E., Rizzi, S., Rocchetti, M., Sola, F., Moll, J., 2012. NMS-P937, an orally available, specific small-molecule polo-like kinase 1 inhibitor with antitumor activity in solid and hematologic malignancies. Mol. Canc. Therapeut. 11, 1006-1016.
Wang, Y., Gratzke, C., Tamalunas, A., Rutz, B., Ciotkowska, A., Strittmatter, F., Herlemann, A., Janich, S., Waidelich, R., Liu, C., Stief, C.G., Hennenberg, M., 2016. Smooth muscle contraction and growth of stromal cells in the human prostate are both inhibited by the Src family kinase inhibitors, AZM475271 and PP2. Br. J. Pharmacol.
Wang, Y., Gratzke, C., Tamalunas, A., Rutz, B., Ciotkowska, A., Strittmatter, F., Herlemann, A., Janich, S., Waidelich, R., Liu, C., Stief, C.G., Hennenberg, M., 2016. Smooth muscle contraction and growth of stromal cells in the human prostate are both inhibited by the Src family kinase inhibitors, AZM475271 and PP2. Br. J. Pharmacol. 173, 3342-3358.
Wang, Y., Gratzke, C., Tamalunas, A., Wiemer, N., Ciotkowska, A., Rutz, B., Waidelich, R., Strittmatter, F., Liu, C., Stief, C.G., Hennenberg, M., 2016. P21-Activated kinase inhibitors FRAX486 and IPA3: inhibition of prostate stromal cell growth and effects on smooth muscle contraction in the human prostate. PloS One 11, e0153312.
Wang, Y., Kunit, T., Ciotkowska, A., Rutz, B., Schreiber, A., Strittmatter, F., Waidelich, R., Liu, C., Stief, C.G., Gratzke, C., Hennenberg, M., 2015. Inhibition of prostate smooth muscle contraction and prostate stromal cell growth by the inhibitors of Rac, NSC23766 and EHT1864. Br. J. Pharmacol. 172, 2905-2917.
Wang Z, Xiao X, Ge R, Li J, Johnson CW, Rassoulian C, et al. (2017) Metformin inhibits the proliferation of benign prostatic epithelial cells. PLoS ONE 12(3): e0173335.
Webber, M.M., Trakul, N., Thraves, P.S., Bello-DeOcampo, D., Chu, W.W., Storto, P.D., Huard, T.K., Rhim, J.S., Williams, D.E., 1999. A human prostatic stromal myofibroblast cell line WPMY-1: a model for stromal-epithelial interactions in prostatic neoplasia. Carcinogenesis 20, 1185-1192.
Weiss, G.J., Jameson, G., Von Hoff, D.D., Valsasina, B., Davite, C., Di Giulio, C., Fiorentini, F., Alzani, R., Carpinelli, P., Di Sanzo, A., Galvani, A., Isacchi, A., Ramanathan, R.K., 2018. Phase I dose escalation study of NMS-1286937, an orally available Polo-Like Kinase 1 inhibitor, in patients with advanced or metastatic solid tumors. Invest. N. Drugs 36, 85-95.
White, C.W., Short, J.L., Evans, R.J., Ventura, S., 2015. Development of a P2X1-purinoceptor mediated contractile response in the aged mouse prostate gland through slowing down of ATP breakdown. Neurourol. Urodyn. 34, 292-298.
Xu, Y., Ventura, S., 2010. Extracts of bark from the traditional Chinese herb Phellodendron amurense inhibit contractility of the isolated rat prostate gland. J. Ethnopharmacol. 127, 196-199.
Yu, Q., Gratzke, C., Wang, R., Li, B., Kuppermann, P., Herlemann, A., Tamalunas, A., Wang, Y., Rutz, B., Ciotkowska, A., Wang, X., Strittmatter, F., Waidelich, R., Stief, C.G., Hennenberg, M., 2019. A NAV2729-sensitive mechanism promotes adrenergic smooth muscle contraction and growth of stromal cells in the human prostate. J. Biol. Chem. 294, 12231-12249.
Yu, Q., Gratzke, C., Wang, Y., Herlemann, A., Strittmatter, F., Rutz, B., Stief, C.G., Hennenberg, M., 2018. Inhibition of prostatic smooth muscle contraction by the inhibitor of G protein-coupled receptor kinase 2/3, CMPD101. Eur. J. Pharmacol. 831, 9-19.
Yu, Q., Gratzke, C., Wang, Y., Wang, X., Li, B., Strittmatter, F., Herlemann, A., Wang, R., Tamalunas, A., Waidelich, R., Stief, C.G., Hennenberg, M., 2019. New strategies for inhibition of non-adrenergic prostate smooth muscle contraction by pharmacologic intervention. Prostate 79, 746-756.
Zhang, Z., Hou, X., Shao, C., Li, J., Cheng, J.X., Kuang, S., Ahmad, N., Ratliff, T., Liu, X., 2014. Plk1 inhibition enhances the efficacy of androgen signaling blockade in castration-resistant prostate cancer. Canc. Res. 74, 6635-6647.

In view of the above, it will be seen that several objectives of the invention are achieved and other advantages attained.

As various changes could be made in the above methods and compositions without departing from the scope of the invention, it is intended that all matter contained in the above description and shown in the accompanying drawings shall be interpreted as illustrative and not in a limiting sense.

As used herein, the singular forms "a", "an" and "the" are intended to include the plural forms as well, unless the context clearly indicates otherwise. Additionally, the use of "or" is intended to include "and/or", unless the context clearly indicates otherwise.

As used herein, in particular embodiments, the terms "about" or "approximately" when preceding a numerical value indicates the value plus or minus a range of 10%. Where a range of values is provided, it is understood that each intervening value, to the tenth of the unit of the lower limit unless the context clearly dictates otherwise, between the upper and lower limit of that range and any other stated or intervening value in that stated range is encompassed within the disclosure. That the upper and lower limits of these smaller ranges can independently be included in the smaller ranges is also encompassed within the disclosure, subject to any specifically excluded limit in the stated range. Where the stated range includes one or both of the limits, ranges excluding either or both of those included limits are also included in the disclosure.

The phrase "and/or," as used herein in the specification and in the embodiments, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements can optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the embodiments, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the embodiments, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the embodiments, shall have its ordinary meaning as used in the field of patent law.

As used herein in the specification and in the embodiments, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements can optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

## Claims

1. Onvansertib for use in a method of treating benign prostatic hyperplasia (BPH) in a patient, the method comprising treating the patient with (a) onvansertib and (b) an α1-blocker.

2. Onvansertib for use according to claim 1, wherein the α1-blocker is alfuzosin, doxazosin, tamsulosin or silodosin.

3. Onvansertib for use according to claim 1, wherein the patient is treated with onvansertib at a dosage of greater than 6 mg/m²/day and less than 50 mg/m²/day. mg/m²/day.

4. Onvansertib for use according to claim 1, wherein the patient is treated with onvansertib concurrently, before or after treatment with an α1-blocker.

5. Onvansertib for use according to claim 1, wherein the patient is treated with onvansertib and an α1-blocker on a different or the same schedule.

## Patentansprüche

1. Onvansertib für die Verwendung in einem Verfahren zum Behandeln einer benignen Prostatahyperplasie (BPH) bei einem Patienten, wobei das Verfahren das Behandeln des Patienten mit (a) Onvansertib und (b) einem α1-Blocker umfasst.

2. Onvansertib für die Verwendung nach Anspruch 1, wobei der α1-Blocker Alfuzosin, Doxazosin, Tamsulosin oder Silodosin ist.

3. Onvansertib für die Verwendung nach Anspruch 1, wobei der Patient mit Onvansertib in einer Dosierung von mehr als 6 mg/m²/Tag und weniger als 50 mg/m²/Tag behandelt wird.

4. Onvansertib für die Verwendung nach Anspruch 1, wobei der Patient mit Onvansertib gleichzeitig, vor oder nach der Behandlung mit einem α1-Blocker behandelt wird.

5. Onvansertib für die Verwendung nach Anspruch 1, wobei der Patient mit Onvansertib und einem α1-Blocker nach einem unterschiedlichen oder dem gleichen Zeitplan behandelt wird.

## Revendications

1. Onvansertib destiné à être utilisé dans une méthode de traitement de l'hyperplasie bénigne de la prostate (BPH) chez un patient, la méthode comprenant le traitement du patient par (a) de l'onvansertib et (b) un α1-bloquant.

2. Onvansertib destiné à être utilisé selon la revendication 1, ledit α1-bloquant consistant en l'alfuzosine, la doxazosine, la tamsulosine ou la silodosine.

3. Onvansertib destiné à être utilisé selon la revendication 1, ledit patient étant traité par onvansertib à une dose supérieure à 6 mg/m²/jour et inférieure à 50 mg/m²/jour.

4. Onvansertib destiné à être utilisé selon la revendication 1, ledit patient étant traité par onvansertib simultanément, avant ou après le traitement par α1-bloquant.

5. Onvansertib destiné à être utilisé selon la revendication 1, ledit patient étant traité par onvansertib et α1-bloquant selon un programme différent ou identique.
